# FASCICULE DE BREVET EUROPEEN

(11) **EP 1 937 205 B1**
(45) Date de publication et mention de la délivrance du brevet: **09.11.2016**
(21) Numéro de dépôt: 06808192.6
(22) Date de dépôt: 22.09.2006
(51) Int. Cl.: A61P 17/16, A61K 8/19, A61K 8/23, A61K 8/25, A61K 8/27, A61K 8/96, A61Q 17/00, A61Q 17/04, A61K 33/00, A61K 33/04, A61K 33/06, A61K 33/08, A61K 33/22, A61K 33/26, A61K 33/30, A61K 33/32, A61K 45/06, A61K 33/34

(54) **COMPOSITION À BASE DE CONCENTRÉS MINÉRAUX DÉRIVÉS DE PIERRES PRÉCIEUSES**
ZUSAMMENSETZUNG AUF BASIS VON MINERALKONZENTRATEN AUS EDELSTEINEN
COMPOSITION BASED ON MINERAL CONCENTRATES DERIVED FOR PRECIOUS STONES

(30) Priorité: 22.09.2005 FR 0509706
(43) Date de publication de la demande: 02.07.2008
(73) Titulaire: Bulgari Parfums SA, 2008 Neuchâtel (CH)
(72) Inventeur: ALLART, Jean-Claude, 62520 Le Touquet (FR); LEFEVRE, Jean-Marie, 80000 Amiens (FR); PEYROT, Jacques, 63000 Clermont-Ferrand (FR); MARTY, Jean-Paul, 83650 Les Adrets de L'Esterel (FR)
(74) Mandataire: L'Helgoualch, Jean
(86) Numéro de dépôt international: PCT/FR2006/002174
(87) Numéro de publication internationale: WO 2007/034088

(56) Documents cités:
- WO-A-99/48505
- WO-A-2005/044185
- DE-A1- 19 541 735
- FR-A- 2 805 743
- US-A- 5 985 021
- US-A1- 2002 012 681
- PATENT ABSTRACTS OF JAPAN vol. 1998, no. 10, 31 août 1998 (1998-08-31) & JP 10 120520 A (MATSUBARA TERUKO; SAMMY:KK), 12 mai 1998 (1998-05-12)

## Description

La présente invention concerne une nouvelle composition utilisable en cosmétologie et en dermatologie, et plus particulièrement une nouvelle composition à base de concentrés minéraux dérivés de pierres précieuses.

La peau constitue un organe essentiel à la vie, qui remplit plusieurs fonctions dont la qualité a une influence déterminante sur la santé de tout individu. Plus particulièrement, la peau constitue une véritable barrière vis-à-vis de l'environnement et elle est soumise à des agressions incessantes. La qualité de ses défenses est donc un élément majeur pour l'efficacité de sa protection.

La peau comprend plusieurs couches intégrées, à savoir la couche superficielle (l'épiderme), et les couches plus profondes (le derme et l'hypoderme), et chacune de ces couches possède des propriétés spécifiques permettant à l'ensemble de réagir et de s'adapter aux conditions de son environnement.

L'épiderme est principalement composé de kératinocytes (90% des cellules épidermiques), de mélanocytes (2 à 3% des cellules épidermiques) et des cellules de Langerhans. Son épaisseur est variable selon les différentes parties du corps.

Le derme est plus épais, et se compose principalement de collagène, d'élastine et de protéoglycanes. Ces trois types de molécules sont synthétisés par les fibroblastes dermiques. Les fibres de collagène assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme est la couche la plus profonde de la peau, et contient les adipocytes qui produisent des lipides afin que le tissu sous-cutané puisse fabriquer une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur ou par les modes de vie. L'apparition de marques pigmentaires, la diminution de l'épaisseur de la peau et son affaissement sont également des changements observés au cours du vieillissement. On sait que la capacité de la peau à remplacer le collagène endommagé diminue avec le temps, et en conséquence des espaces et des irrégularités apparaissent dans le réseau du collagène.

Ainsi les fonctions de la peau sont constamment sollicitées, et notamment l'épiderme par sa fonction de kératinisation, le derme par sa fonction fibroblastique, la jonction dermo-épidermique par sa fonction d'ancrage et sa fonction régulatrice, ainsi que la microcirculation par sa fonction nutritionnelle et oxygénatrice.

La protection de la peau doit porter en priorité sur la surface cutanée car toute perturbation du stratum corneum et du film hydrolipidique de surface entraîne des effets sur les structures plus profondes de la peau. La vascularisation et la microcirculation doivent aussi être prises en considération pour assurer le maintien d'un bon équilibre cutané.

On connaît de nombreuses compositions destinées à protéger la peau contre les effets néfastes du rayonnement ultraviolet, et ces compositions peuvent contenir des filtres ou des pigments formant un écran solaire. Par exemple on peut utiliser des filtres solaires UV-A et UV-B hydrophiles ou lipophiles, qui peuvent être choisis parmi la benzophénone ou un dérivé de benzophénone tel que la 2-hydroxy-4-méthoxybenzophénone (Eusolex® 4360), ou un ester d'acide cinnamique tel que le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), et le 4-méthoxydibenzoylméthane. Ces filtres peuvent être utilisés isolément ou une combinaison, comme dans les brevet EP 514.491 et EP 685.225. On peut aussi utiliser des pigments formant écran anti-ultraviolet, qui peuvent par exemple être choisis parmi le dioxyde de titane, l'oxyde de zinc, de zirconium ou encore d'aluminium. On peut en particulier utiliser des nanopigments d'oxydes métalliques de taille de particules comprise entre 5 et 100 nm, tels que ceux décrits dans la demande de brevet EP 518.773.

Divers traitements ont aussi été proposés pour protéger la peau et atténuer les signes du vieillissement cutané. Par exemple, on a proposé des traitements à base de compositions telles que crèmes et lotions contenant des alpha-hydroxyacides ou des rétinoïdes, appliquées régulièrement, pour réduire progressivement le nombre de rides et ridules.

Toutefois, si tous ces produits et méthodes connus peuvent avoir un effet favorable sur les signes du vieillissement cutané, par exemple en protégeant la peau, ou en masquant ou réduisant les rides, ils n'ont généralement qu'un effet limité sur l'évolution qui aboutit à ces signes du vieillissement.

Par ailleurs, on connaît aussi quelques compositions cosmétiques comprenant des composés minéraux. Par exemple, le brevet US 4.857.306 décrit des compositions destinées au maquillage qui peuvent contenir des poudres à base de pierres précieuses colorées dans un support liquide à forte viscosité. Toutefois, ces divers composés minéraux dérivés de pierres précieuses ont été utilisés dans des compositions en raison de leurs propriétés colorées ou granulométriques. Par contre, aucune propriété de protection de la peau n'a été mise en évidence. La demande WO 2005/044185 décrit une composition destinée à protéger la peau contre les émissions électromagnétiques contenant une combinaison d'un composé cationique, d'un composé minéral tel que fuchsite ou malachite et de particules métalliques. Le brevet FR 2805743 décrit l'utilisation de poudre de jade de néphrite dans une composition en raison de ses effets médicaux connus. Le brevet US 5.985.021 décrit l'utilisation de silimanite (silicate d'alumine), de topaze, de zéolite et d'hématite dans des savons et compositions cosmétiques en raison des effets bénéfiques qu'ils sont supposés avoir sur la peau. La demande de brevet US 2002/012681 concerne une composition cosmétique comprenant des poudres minérales ayant des propriétés de fluorescence. Cependant, ces compositions ne permettent pas de procurer à la fois une protection efficace contre les rayonnements et une activation des fonctions microcirculatoires de la peau.

Il est donc apparu souhaitable de mettre au point des compositions cosmétiques ou dermatologiques susceptibles de procurer une excellente protection superficielle de la peau et plus particulièrement une composition procurant
- une protection contre les effets nocifs des rayonnements électromagnétiques et contre les agressions physiques et chimiques de l'environnement afin de préserver ses défenses naturelles, de maintenir une bonne hydratation locale, de préserver sa fonction fibroblastique,
- une activation des fonctions microcirculatoires afin d'améliorer la nutrition, le drainage et l'oxygénation de la peau.

La présente invention a donc pour objet une composition utile en cosmétologie et en dermatologie assurant la protection de la peau, notamment contre les rayonnements électromagnétiques et les agressions de l'environnement, favorisant la microcirculation cutanée et susceptible de maintenir son hydratation.

La composition suivant la présente invention, utile en cosmétique et en dermatologie pour assurer la protection de la peau comme indiqué ci-dessus, est telle que définie dans l'objet de la revendication 1.

Plus particulièrement, la composition comprend d'une part une poudre ou un mélange de poudres sèches obtenues à partir de pierres précieuses ou semi-précieuses contenant au moins un dérivé du silicium et d'autre part une suspension aqueuse de poudres obtenues à partir de pierres précieuses ou semi-précieuses contenant au moins un oligoélément autre que le silicium, pour former un concentré minéral, en mélange avec un support et des excipients physiologiquement acceptables.

La composition suivant la présente invention comprend une poudre ou un mélange de poudres contenant d'une part un oxyde silicium (silice) et/ou un silicate, et d'autre part un ou plusieurs oligoéléments, autres que le silicium.

Les oligoéléments sont choisis parmi le fer, le cuivre, le zinc, le manganèse, le molybdène, le bore, le cobalt, l'aluminium et le sélénium.

Ces poudres et mélanges de poudres à base de dérivé de silicium et/ou de silicate, et d'oligoéléments, sont obtenues de préférence à partir de pierres précieuses ou semi-précieuses et plus particulièrement de tourmaline, d'aigue-marine, d'émeraude, de citrine, d'améthyste, de malachite, de rhodocrosite, de smithsonite, de rhodolite, de lapis lazuli, de saphir, de rubis et de topaze.

On choisit de préférence des pierres précieuses ou semi-précieuses contenant du silicium ou des silicates, et par exemple la tourmaline qui contient du borosilicate, l'aigue-marine et l'émeraude qui contiennent un aluminosilicate, la citrine et l'améthyste qui contiennent du dioxyde de silicium. Les pierres précieuses ou semi-précieuses contenant des oligoéléments sont de préférence choisies parmi la malachite qui contient du cuivre, la rhodocrosite qui contient du manganèse, le saphir qui contient de l'aluminium, et la smithsonite qui contient du zinc.

Ainsi, suivant une forme préférentielle de réalisation de l'invention, la composition contient d'une part au moins une poudre obtenue à partir de pierres précieuses ou semi-précieuses contenant un dérivé de silicium choisi parmi un borosilicate, un aluminosilicate, et le dioxyde de silicium, et d'autre part au moins une poudre contenant un oligoélément choisi parmi le fer, le cuivre, le zinc et le manganèse. Les pierres précieuses ou semi-précieuses contenant un dérivé de silicium sont choisies de préférence parmi l'aigue-marine, la tourmaline, l'émeraude, la citrine et l'améthyste, tandis que les pierres précieuses contenant un oligoélément sont choisies de préférence parmi la malachite, la rhodocrosite, le saphir et la smithsonite.

Ces pierres précieuses ou semi-précieuses sont disponibles sous forme de poudres et sont réduites en poudre par broyage de manière à obtenir une granulométrie comprise entre 1 µm et 50 µm. La dimension moyenne des particules des poudres est de préférence inférieure à 50 µm, et plus particulièrement inférieure à 20 µm environ.

Par exemple, l'aigue marine est une poudre ultrafine, ayant une granulométrie moyenne inférieure à 10 microns. Sa formule générique comprend un aluminosilicate de béryllium avec du chrome et des traces du magnésium. Son système cristallin est hexagonal avec un arrangement en colonne. Le dosage des métaux lourds est inférieur à 50 ppm.

La citrine est une poudre ultrafine de couleur gris pâle avec une granulométrie moyenne inférieure à 10 microns. Sa formule générique comprend du dioxyde de silicium de fer et de manganèse, calcium et de titane. Son système cristallin est hexagonal à six pans. Sa teneur en métaux lourds est inférieure à 50 ppm

Suivant une forme préférentielle de réalisation, la composition comprend en mélange une ou plusieurs poudres sèches contenant le dérivé de silicium et une suspension aqueuse comprenant une ou plusieurs poudres contenant les oligoéléments, pour former un concentré minéral.

Le rapport en poids poudres sèches / suspension de poudres peut être compris entre 1/5 et 1/1 environ.

Ainsi, suivant une forme préférentielle de réalisation de l'invention, la composition comprend un mélange de poudres sèches obtenues par broyage d'aigue-marine, de tourmaline, de citrine et d'améthyste, et d'une suspension aqueuse de poudres obtenues par broyage de malachite, de rhodocrosite et de smithsonite, ainsi qu'un support et des excipients physiologiquement acceptables.

Par "physiologiquement acceptable" au sens de la présente invention, on entend des supports et excipients d'un type couramment utilisé dans les compositions cosmétiques et dermatologiques, neutres vis-à-vis des principes actifs utilisés, ne présentant pas d'effet toxique et n'occasionnant aucun effet secondaire néfaste sur la peau.

Suivant une variante, il est avantageux d'ajouter à la composition ci-dessus du saphir sous forme de poudre, en une quantité représentant entre 1 et 20% en poids de la composition, et de préférence entre 5 et 15% en poids. La taille moyenne des particules peut être comprise entre 5 et 20 µm. Ajouté sous forme de poudre, le saphir améliore sensiblement le pouvoir de réflexion du rayonnement ultraviolet et augmente ainsi le pouvoir de protection solaire des compositions suivant l'invention.

Les poudres contenant des oligoéléments tels que le fer, le zinc, le cuivre, et le manganèse, peuvent être généralement utilisées sous forme de suspensions ou d'extraits aqueux disponibles dans le commerce. Ces suspensions ou extraits aqueux sont solubles dans l'eau et généralement insolubles dans les huiles minérales et végétales. Leur extrait sec est généralement compris entre 0,2% et 10% et leur teneur en oligoélément comprise entre 1 et 15%, voire davantage. La concentration d'utilisation de ces suspensions ou extraits aqueux peut être de l'ordre de 0,5% à 50% et de préférence de 1% à 30%.

Il peut être avantageux d'incorporer dans la composition une substance améliorant le pouvoir couvrant, comme par exemple le nitrure de bore. Cette substance est ajoutée après mélange des poudres sèches et des poudres en suspension.

Un liant tel qu'un polyol, un polyéthylène glycol, le butylène glycol et la glycérine peut être avantageusement incorporé dans la composition du concentré minéral. Suivant l'invention, on utilise de préférence la glycérine.

Un gélifiant est de préférence ajouté pour améliorer les propriétés physiques de la composition. On peut utiliser plus particulièrement un gélifiant choisi parmi les polyacrylamides (par exemple du type Carbopol), les copolymères acrylate/acide acrylique ou acrylamide/acide acrylamido propane sulfonique, des mélanges à base de polyacrylamides, et par exemple un mélange de polyacrylamide, C13-14-isoparaffine et Laureth-7 (Sepigel 305®, de la société Seppic), les dérivés de cellulose comme l'hydroxypropyl cellulose, le chitosan, des mucopolysaccharides végétaux, et les argiles.

Le concentré minéral contenant le cas échéant le nitrure de bore est dilué dans le gélifiant, et les excipients sont ajoutés de telle sorte que la teneur du concentré minéral dans la composition finale soit comprise entre 1% et 15%, de préférence entre 2% et 10% en poids par rapport au poids total de la composition.

Les essais effectués avec divers concentrés minéraux suivant l'invention ont mis en évidence d'intéressantes propriétés utilisables en cosmétologie et en dermatologie, notamment des effets de protection directe de la peau, en particulier un effet de protection solaire, un effet anti-oxydant, et un effet thermique et microcirculatoire.

### Protection solaire

Des essais ont été effectués avec des concentrés minéraux dilués à 2,5%, 5% et 10% respectivement, dans un véhicule neutre pouvant contenir le gélifiant (Sepigel 305) éventuellement additionné de liant, pour évaluer le pouvoir de protection contre les rayonnements, par comparaison avec un écran solaire connu à base d'oxyde de titane, et ont mis en évidence une excellente efficacité dans un domaine de longueurs d'onde allant de 190 à 900 nm, c'est-à-dire un domaine allant de l'ultraviolet à l'infrarouge.

Il est particulièrement intéressant d'observer que les résultats sont sensiblement équivalents aux trois concentrations testées de 2,5%, 5% et 10%, ce qui montre qu'un effet protecteur satisfaisant contre les rayonnements est obtenu dès la dose de 2,5% et qu'au-delà de cette valeur, l'effet ne dépend plus de la dose.

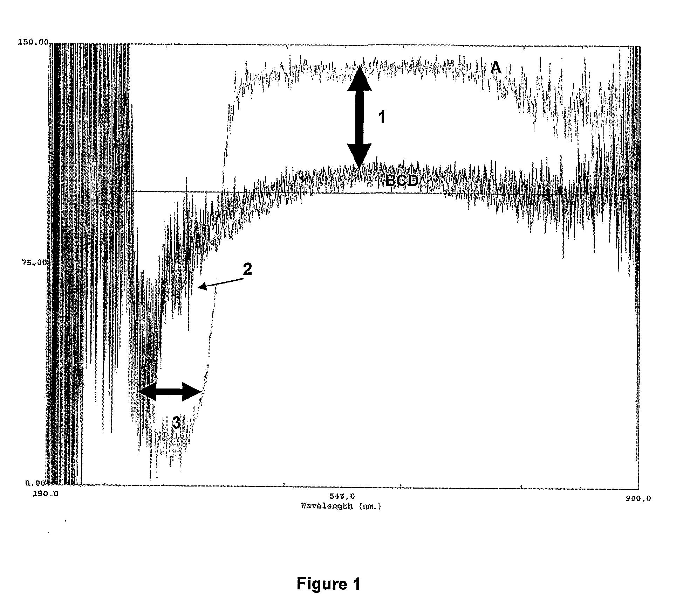

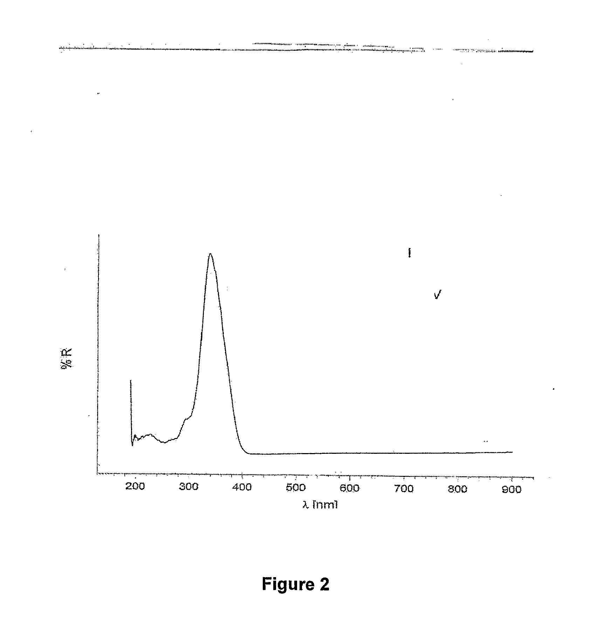

Plus particulièrement, en utilisant le concentré minéral ayant la composition A indiquée dans l'Exemple 1, on a vérifié par spectroscopie l'absorption et la réflexion de l'UV à l'IR par comparaison avec l'oxyde de titane. Les spectres sont représentés sur les Figures 1 et 2 jointes.

La Figure 1 montre le spectre d'absorption dans une gamme de longueurs d'onde allant de 190 nm à 900 nm indiquée en abscisse. La courbe A correspond à l'oxyde de titane (TiO₂) pris comme référence, tandis que les courbes B, C et D correspondent au concentré minéral A de l'exemple 1, aux concentrations de 10%, 5% et 2,5% respectivement. Ces trois courbes sont pratiquement superposées, ce qui montre que l'effet d'absorption ne dépend pas de la dose au-delà de 2,5%. L'écart (1) entre la courbe A et les courbes B, C et D, montre que le concentré minéral de l'invention présente une plus forte absorption que l'oxyde de titane. La partie (2) des courbes montre que le concentré minéral présente une absorption équivalente à celle de l'oxyde de titane dans l'ultraviolet, et l'écart (3) montre que l'absorption du titane est plus large dans cette zone.

La Figure 2 montre le spectre de réflexion du concentré minéral B décrit dans l'Exemple 1 ci-après par comparaison avec l'oxyde de titane (TiO₂) pris comme référence. Ce spectre montre que dans la zone de l'ultraviolet, le concentré minéral de l'invention possède un indice de réflexion supérieur à celui de l'oxyde de titane.

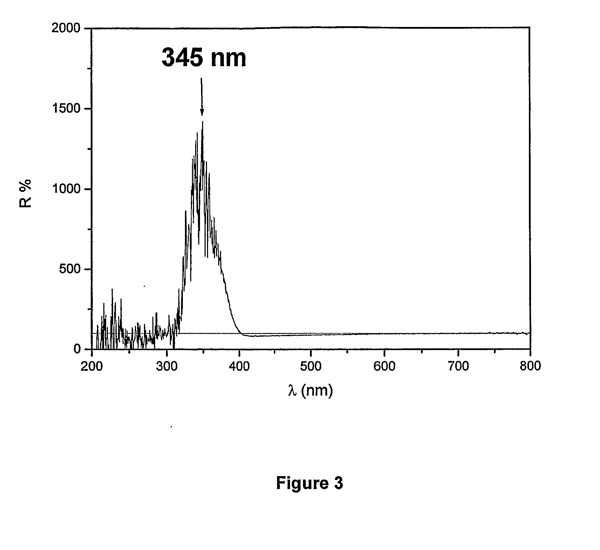

La Figure 3 montre le spectre de réflexion de la composition correspondant au concentré minéral F décrit dans l'Exemple 1, l'oxyde de titane étant pris comme référence. Ce spectre montre clairement une très nette augmentation du pouvoir de réflexion dans le domaine de l'ultraviolet centré sur 345 nm.

Ces résultats montrent que les compositions suivant l'invention procurent une protection efficace contre les rayonnements, en particulier les rayons UV, sans qu'il soit nécessaire d'ajouter un filtre UV ou un écran solaire.

### Protection directe

L'effet protecteur immédiat a été vérifié par un test de picotement ("stinging test"). Ce test consiste à provoquer une irritation en appliquant une goutte d'acide lactique à 10% sur l'un des plis du nez d'un patient, puis à comparer avec un placebo sur l'autre pli du nez. Le placebo utilisé est du sérum physiologique. La composition de l'invention a été préalablement appliquée sur la peau, avant l'application de l'acide lactique et du sérum physiologique, chez la moitié des patients. La composition de l'invention est le concentré minéral A de l'Exemple 1 dilué à 2,5% dans le Sepigel 305. Le test est effectué sur un échantillon de 10 patients présentant une peau fine et sensible.

Le degré d'irritation est évalué sur une échelle de 0 (aucune irritation) à 10 (irritation importante). La moyenne des patients n'ayant pas reçu la composition de l'invention est de 7,5 tandis qu'elle n'est que de 3,5 chez les patients qui l'on reçue.

Ce test montre l'effet protecteur du concentré minéral suivant la présente invention.

### Effet nanti-oxydant

Le concentré minéral suivant l'invention a montré un effet anti-oxydant inattendu mesuré par des tests in vivo.

L'évaluation de l'effet anti-oxydant a été faite par mesure de la dégradation du squalène sous rayonnement ultraviolet. Le squalène est un composé naturel présent dans le sébum de la peau, qui, en raison de sa structure chimique insaturée, est facilement oxydé après exposition aux ultraviolets. Le produit d'oxydation peut être dosé par chromatographie HPLC. Le principe du test consiste donc à mesurer l'intensité du pic de HPLC caractéristique du squalène et de son produit d'oxydation (monohydroperoxyde), après exposition contrôlée aux ultraviolets.

Le protocole d'évaluation consiste à appliquer sur la peau les concentrés minéraux A et B de l'invention décrits dans l'Exemple 1, puis à prélever un échantillon de sébum sur le front de chaque volontaire au moyen d'une bande de Sebutape® après 1 heure de contact avec le produit, puis irradiation aux ultraviolets (lampe UVA-B Philips, délivrant 30-40 joules/cm², placée à 21 cm pendant 16 minutes) afin d'oxyder le squalène présent dans le sébum prélevé.

La surface de la peau a été nettoyée 5 minutes avant le début du test au moyen d'un coton imbibé d'alcool éthylique à 70° de manière à éliminer les lipides présents en surface. Deux échantillons de sébum ont été prélevés sur chaque volontaire participant au test au moyen de deux bandes de Sebutape® et chaque bande est divisée en deux parties, dont l'une est exposée aux UV et l'autre ne l'est pas pour servir de référence. Les mêmes tests ont été effectués avec un placebo (même composition sans le concentré minéral de l'invention).

Les valeurs moyennes de quantités de squalène non oxydé détectées dans le sébum pour les concentrés minéraux A et B suivant l'invention, à T0 (immédiatement après application de la composition) et à T1 (1 heure après application), sont indiquées dans le tableau suivant (quantités en µg dans la bande de Sebutape®) :

| | T0 | T1 |
|---|---|---|
| Concentré minéral A non irradié | 54,45 ± 48,93 | 56,67 ± 21, 34 |
| Concentré minéral A irradié | 10,68 ± 10,53 | 14,56 ± 7,61 |
| Concentré minéral B non irradié | 45,64 ± 26,11 | 64,48 ± 28,64 |
| Concentré minéral B irradié | 11,04 ± 9,69 | 21,17 ± 11,37 |

En se fondant sur une valeur 100 pour l'échantillon non irradié, les quantités relatives de squalène non oxydé sont exprimées dans lé tableau ci-après :

| | T0 | T1 |
|---|---|---|
| Concentré minéral A | 19% | 26% |
| Concentré minéral B | 26% | 33% |

Ces résultats montrent l'efficacité du concentré minéral de l'invention contre l'oxydation du squalène par exposition aux ultraviolets. L'application d'une composition suivant l'invention sur la peau avant exposition aux ultraviolets induit un effet protecteur du sébum.

De plus, les essais montrent que la composition de l'invention n'a pas d'effet oxydant, même après exposition, contrairement à certaines huiles dont les produits de dégradation sont oxydants et ont un effet nocif pour la peau, en particulier des huiles de monoï ou certaines benzophénones utilisés comme produits de protection solaire. Ce résultat est d'autant plus intéressant que la composition de l'invention procure une protection contre le rayonnement ultraviolet, comme indiqué plus haut, qui rend alors moins utile l'addition de filtres UV.

En outre, on a constaté que le concentré minéral de l'invention ne modifie pas l'excrétion du sébum et n'a donc aucun effet occlusif, et qu'il peut même avoir un effet d'absorption du sébum.

### Effet sur la microcirculation cutanée

L'étude a été faite sur un échantillon de 5 femmes d'âge compris entre 23 et 32 ans.

Les moyennes des résultats sont regroupées au tableau ci-dessous. Une première mesure est faite au moment de l'application du concentré minéral A sur la peau (T0) et une deuxième mesure est faite 5 minutes plus tard (T1). Une seule application de 50 mg de concentré minéral est faite sur la peau de la joue et de la pommette d'une face du visage. Les mesures de thermographie cutanée sont faites au moyen d'une caméra placée à 62 cm du visage du patient. La mesure du flux circulatoire est faite dans l'obscurité au moyen d'un laser Doppler.

| Valeurs moyennes | T0 | T1 |
|---|---|---|
| Thermographe | 29,65 | 29,26 |
| Laser (flux) | 396,16 | 397,52 |
| Thermocouple | 0,65 | 0,45 |

Cette étude montre que l'application du concentré minéral A décrit dans l'Exemple 1, sur la peau, entraîne des modifications thermiques constatées par thermographie et par thermocouple. Ces modifications sont constatées 5 minutes seulement après application sur la peau.

La diminution thermique observée (thermographe et thermocouple) prouve que le concentré minéral de l'invention exerce très rapidement un effet bouclier en diminuant les pertes caloriques à la surface de la peau, sans réduction du flux circulatoire mesuré par laser Doppler. Ce résultat est inattendu car on aurait pu prévoir, selon les règles classiques de la thermorégulation liée à la microcirculation, une réduction du flux circulatoire. Au contraire, la légère augmentation du flux constatée traduit une action énergétique sur la vasomotricité induite par le concentré minéral de l'invention.

Ces différents tests montrent que le concentré minéral suivant l'invention procure un effet protecteur contre les rayonnements dans un domaine de longueurs d'onde allant de l'ultraviolet à l'infrarouge, contre les irritations de la peau, une protection antioxydante, avec un effet thermique de surface, une stimulation de la microcirculation, un pouvoir absorbant du sébum sans effet occlusif, ainsi qu'une tolérance parfaite de la peau vis-à-vis du concentré minéral sans phénomène de sensibilisation.

Ce concentré minéral constitue un principe actif présentant des propriétés utiles permettant de l'incorporer dans des compositions topiques à usage dermatologique ou cosmétologique, dans une concentration qui peut être comprise entre 1 et 50% en poids par rapport au poids total de la composition, de préférence entre 1 et 20% et plus préférentiellement entre 2 à 10% dans le but de protéger la peau ou de renforcer ses défenses naturelles.

Suivant une forme de réalisation de la présente invention, il peut être avantageux d'incorporer dans la composition une substance procurant une activité complémentaire utile, par exemple pour favoriser la synthèse du collagène, et plus particulièrement un lipopeptide tel que le palmitoyl-lysyl-thréonyl-thréonyl-lysyl-sérine (Matrixyl® de la société Sederma), généralement sous forme de solution hydro-alcoolique.

Le concentré minéral peut aussi être utilisé pour renforcer l'action d'autres actifs présents dans de telles compositions topiques pour favoriser l'hydratation de la peau, la protection solaire, la lutte contre les phénomènes inflammatoires, la lutte contre le vieillissement cutané et plus généralement le maintien d'une bonne homéostasie.

L'agent hydratant ou humectant éventuellement incorporé dans la composition peut être choisi parmi les agents hydratants utilisés classiquement dans les compositions cosmétiques ou dermatologiques, et par exemple un polyol, la glycérine (glycérol et dérivés de glycérol), la diglycérine, le polyéthylène glycol, le sorbitol, les polyacrylates et polyméthacrylates de glycéryle, les mucopolysaccharides tels que l'acide hyaluronique, des dérivés du chitosan et des dérivés de l'acide pyrrolidone carboxylique. La teneur en agent hydratant ou humectant est généralement comprise entre 0,1 et 10% en poids par rapport au poids total de la composition.

Les conservateurs usuels de la technique des compositions dermatologiques ou cosmétologiques peuvent être utilisés dans l'invention, et par exemple un alcool tel que l'éthanol, l'isopropanol et le phénoxy-éthanol, l'acide benzoïque et un p-hydroxybenzoate d'alkyle tel que les p-hydroxy-benzoates de méthyle et de propyle (Méthylparaben et Propylparaben), ou encore la chlorphénésine ou l'imidazolidinyl urée. Ces conservateurs peuvent être utilisés isolément ou en combinaison.

La composition peut être complétée par divers excipients selon la nature des phases désirées, tels que le triglycéride caprylique / caprate C8-C10 (Estasan ou Miglyol 812), ou l'acide oléique, comme constituants de la phase grasse dans le cas d'une émulsion, ou du cyclopentasiloxane pour améliorer les propriétés de toucher de la composition.

Les compositions suivant l'invention peuvent se présenter sous forme de crèmes, baumes, émulsions huile-dans-eau (H/E) ou émulsions eau-dans-huile (E/H), gels, sérums, masques, onguents ou pommades, et de préférence sous forme de crèmes ou émulsions eau dans silicone ou huile dans eau. Ces diverses formes galéniques sont préparées suivant les techniques usuelles. Ces compositions peuvent être présentées sous forme de complément de maquillage ou de produits de soins, par exemple de baume pour les lèvres.

Les exemples suivants illustrent plus en détail l'invention. Dans ces exemples de composition, les parties sont exprimées en poids, sauf indication contraire.

### Exemple 1

### Préparation d'un concentré minéral.

Dans un récipient de 1 l équipé d'un mélangeur, on verse successivement les poudres suivante :

| | |
|---|---|
| aigue-marine (dimension moyenne 10 µm) | 2,5 g |
| tourmaline (dimension moyenne 10 µm) | 15,0 g |
| citrine (dimension moyenne 10 µm) | 7,5 g |

Après avoir soigneusement mélangé les poudres pendant 15 minutes environ, on introduit les extraits aqueux indiqués ci-dessous :

| | |
|---|---|
| Malakite® (extrait aqueux à 5,0 g/l de cuivre) | 15,0 g |
| Rhodolite® (extrait aqueux à 3,0 g/l de manganèse) | 30,0 g |

La Malakite® est un extrait aqueux de malachite disponible dans le commerce (Gattefossé) ayant une teneur en cuivre supérieure à 5 g/l et dont l'extrait sec est compris entre 5 et 7%. La Rhodolite® est un extrait aqueux de rhodocrosite disponible dans le commerce (Gattefossé) ayant une teneur en manganèse comprise entre 1,0 et 3,5 g/l et dont l'extrait sec est compris entre 0,7 et 1,5%.

Le mélange obtenu est maintenu sous agitation à température ambiante pendant environ 15 minutes, pour former un concentré minéral, puis on ajoute un gélifiant constitué par 2,0 g de mélange de polyacrylamide, C13-14-isoparaffine et Laureth-7 (Sepigel 305®) additionné de 16,5 g de nitrure de bore (poudre blanche de dimension moyenne de grains égale à 2 µm).

On obtient ainsi un concentré minéral A additionné de gélifiant et de nitrure de bore ayant la composition pondérale ci-dessous exprimée en parties en poids :

### Concentré minéral A

| | |
|---|---|
| aigue-marine | 2,5 |
| tourmaline | 15,0 |
| citrine | 7,5 |
| Malakite® | 15,0 |
| Rhodolite® | 30,0 |
| glycérine | 11,5 |
| gélifiant | 2,0 |
| nitrure de bore | 16,5 |

Le gélifiant utilisé est le Sepigel 305 comme pour le concentré minéral précédent.

### Concentré minéral B

En procédant de la même manière, on prépare un concentré minéral B additionné de gélifiant et de nitrure de bore ayant la composition ci-dessous exprimée en parties en poids :

| | |
|---|---|
| aigue-marine | 2,5 |
| tourmaline | 20,0 |
| citrine | 10,0 |
| Malakite® | 7,5 |
| Rhodolite® | 15,0 |
| glycérine | 20,0 |
| nitrure de bore | 25,0 |

La glycérine est utilisée pour absorber l'eau et épaissir la composition.

### Concentré minéral C

En procédant de la même manière que ci-dessus, on prépare le concentré minéral suivant :

| | |
|---|---|
| aigue-marine | 7,0 |
| tourmaline | 15,0 |
| améthyste | 3,0 |
| Malakite® | 25,0 |
| Rhodolite® | 25,0 |
| glycérine | 8,0 |
| gélifiant | 2,0 |
| nitrure de bore | 15,0 |

### Concentré minéral D

| | |
|---|---|
| aigue-marine | 5,0 |
| tourmaline | 15,0 |
| citrine | 5,0 |
| Malakite® | 22,5 |
| Rhodolite® | 22,5 |
| glycérine | 11,0 |
| gélifiant | 2,5 |
| nitrure de bore | 16,5 |

### Concentré minéral E

| | |
|---|---|
| aigue-marine | 5,0 |
| tourmaline | 15,0 |
| citrine | 5,0 |
| Malakite® | 22,5 |
| Zin'cite® | 22,5 |
| glycérine | 11,0 |
| gélifiant | 2,5 |
| nitrure de bore | 16,5 |

La Zin'cite® est un extrait aqueux de smithsonite disponible dans le commerce (Gattefossé) ayant une teneur en zinc comprise entre 1,2 et 2,5 g/l et dont l'extrait sec est compris entre 0,4 et 1,0%.

### Concentré minéral F

| | |
|---|---|
| tourmaline | 25,0 |
| citrine | 0,5 |
| Malakite® | 5,0 |
| saphir | 10.0 |
| glycérine | 30,0 |
| nitrure de bore | 25,0 |
| eau | 4,5 |

### Exemple 2

Par les techniques usuelles, on prépare une crème de jour ayant la composition suivante indiquée en parties en poids :

| | |
|---|---|
| Concentré minéral A ci-dessus | 5,00 |
| EDTA | 0,05 |
| PEG 400 | 3,00 |
| Glycérine | 4,00 |
| Phénoxyéthanol | 0,80 |
| Chlorphénésine | 0,10 |
| triglycéride caprylique / caprique | 6,00 |
| Poly-isobutène | 4,00 |
| CETYL ALCOHOL(and) GLYCERYL STEARATE(and) PEG-75 STEARATE (and) CETEH-20 (and) STEARETH-20 | 4,00 |
| Cera alba | 0,50 |
| Huile de noix de macadamia | 1,00 |
| Succinate d'octényle | 1,00 |
| Complexe lipoprotéique | 2,00 |
| Cyclopentasiloxane | 3,00 |
| Acrylate de sodium | 2,00 |
| Eau qsp | 100,00 |

Cette crème est destinée à une utilisation en application sur la peau du visage et du décolleté une à deux fois par jour, pendant 6 à 12 semaines. Elle procure une bonne hydratation et une excellente protection contre les agressions de l'environnement, en particulier contre les rayons ultraviolets.

### Exemple 3

Par les techniques usuelles, on prépare une crème de jour ayant la composition suivante indiquée en parties en poids :

| | |
|---|---|
| Concentré minéral B ci-dessus | 5,00 |
| Propylène glycol | 5,00 |
| Palmitate d'octyle | 2,00 |
| Poly-isobutène | 7,00 |
| Phénoxyéthanol | 0,80 |
| Chlorphénésine | 0,20 |
| triglycéride caprylique / caprique | 6,00 |
| Diméthicone | 0,20 |
| Acrylate / C10-C30 Alkylacrylate crosspolymer | 0,30 |
| Cire d'abeille | 0,50 |
| Acétate de tocophéryle | 0,10 |
| Carbomer | 0,50 |
| Triéthanolamine | 0,60 |
| Matrixyl® | 2,00 |
| Eau | qsp 100,00 |

Cette crème peut être utilisée en application sur la peau du visage une à deux fois par jour, pendant 10 semaines et procure une bonne hydratation et une excellente protection contre les agressions de l'environnement, en particulier contre les rayons ultraviolets.

### Exemple 4

Par les techniques usuelles, on prépare un lait démaquillant ayant la composition suivante indiquée en parties en poids :

| | |
|---|---|
| Concentré minéral A ci-dessus | 5,00 |
| Stéarate de glycérol | 4,50 |
| Acide stéarique | 3,00 |
| Palmitate de cétyle | 0,30 |
| Poly-isobutène | 7,00 |
| Acide sorbique | 0,10 |
| Chlorphénésine | 0,20 |
| triglycéride caprylique / caprique | 3,00 |
| Monopropylène glycol | 3,00 |
| Allantoïne | 0,30 |
| Beurre de karité | 0,80 |
| Acétate de tocophéryle | 0,10 |
| Alginate de sodium | 0,30 |
| Phénoxyéthanol | 0,80 |
| Triéthanolamine | 0,60 |
| Matrixyl® | 2,00 |
| Eau | qsp 100,00 |

Ce lait est utilisé une fois par jour pour le démaquillage de la peau.

### Exemple 5

On prépare un sérum correcteur par les techniques usuelles de fabrication, ayant la composition pondérale suivante :

| | |
|---|---|
| Concentré minéral C ci-dessus | 10,00 |
| Chlorphénésine | 0,20 |
| Carbomer® | 0,45 |
| Glycérine | 10,00 |
| Triéthanolamine | 0,80 |
| Gomme xanthane | 0,20 |
| Matrixyl® | 2,00 |
| Eau | qsp 100,00 |

Ce sérum est appliqué sur la peau le soir en massage sur le visage et le décolleté, jusqu'à complète pénétration, et procure un effet tenseur de la peau.

### Exemple 6

On prépare un sérum traitant contenant le concentré minéral F décrit dans l'Exemple 1 et ayant la composition suivante, suivant une méthode usuelle décrite ci-après.

| | |
|---|---|
| Polysorbate 20® | 2,00 |
| Alkylate / C₁₀₋₃₀ alkyl-acrylate cross polymer (Ultrez 21®) | 0,30 |
| Glycérine | 5,00 |
| Diglycérine | 4,00 |
| EDTA disodique | 0,10 |
| Conservateur | 1,00 |
| Alcool polyvinylique | 0,20 |
| Eau | 20,00 |
| Simulgel NS® | 1,00 |
| Neopentanoate d'isodécyle | 3,00 |
| Ethylhexanoate de cétéaryle | 1,00 |
| Concentré minéral F ci-dessus | 1,00 |
| Plastic Powder D 400® | 3,00 |
| Methyl methacrylate crosspolymer (Micropearl M310®) | 3,00 |
| Cyclopentasiloxane | 7,00 |
| Alcool éthylique 96°2 | 3,00 |
| Matrixyl 3000® | 7,00 |
| Parfum | 0,50 |

L'émulsionnant (Polysorbate 20), le gélifiant (Ultrez 21), l'agent hydratant (glycérine, diglycérine), l'EDTA, l'alcool polyvinylique, les conservateurs (par exemple chlorphénésine et phénoxyéthanol) et l'eau sont mélangés à chaud (40 - 45°C) et on ajoute au mélange le Simulgel, le néopentanoate d'isodécyle et l(éthylhexanoate de cétéaryle, et éventuellement 0,20 parties de soude à 40% jusqu'à obtenir un mélange homogène. Après refroidissement à 30-35°C, sous agitation, on ajoute le concentré minéral F décrit dans l'Exemple 1, puis les autres ingrédients. Le Matrixyl et les parfums sont ajoutés à température ambiante.

## Revendications

1. Composition cosmétique et/ou dermatologique pour la protection de la peau, **caractérisée en ce qu'**elle comprend :
- une poudre ou un mélange de poudres sèches obtenues à partir de pierres précieuses ou semi-précieuses contenant au moins un dérivé du silicium comprenant un oxyde de silicium (silice) et/ou un silicate, lesdites pierres précieuses ou semi-précieuses étant choisies parmi la tourmaline, l'aigue-marine, l'émeraude, la citrine, l'améthyste, le lapis lazuli, et la topaze ; et
- une suspension aqueuse de poudres obtenues à partir de pierres précieuses ou semi-précieuses contenant au moins un oligoélément autre que le silicium, lesdites pierres précieuses ou semi-précieuses étant choisies parmi la malachite, la rhodocrosite, la smithsonite, le rubis, et le saphir ;
pour former un concentré minéral, en mélange avec un support et des excipients physiologiquement acceptables.

2. Composition selon la revendication 1, **caractérisé en ce que** l'oligoélément est choisi parmi le fer, le cuivre, le zinc, le manganèse, le molybdène, le bore, le cobalt, l'aluminium et le sélénium.

3. Composition selon la revendication 1 ou 2, **caractérisé en ce que** le dérivé de silicium est choisi parmi un borosilicate, un aluminosilicate, et le dioxyde de silicium.

4. Composition selon l'une quelconque des revendications précédentes, **caractérisé en ce que** l'oligoélément est choisi parmi le fer, le cuivre, le zinc et le manganèse.

5. Composition selon l'une quelconque des revendications précédentes, **caractérisée en ce que** la granulométrie des poudres est comprise entre 1 µm et 50 µm.

6. Composition selon la revendication 1, **caractérisée en ce que** le mélange de poudres sèches est obtenu par broyage d'aigue-marine, de tourmaline, d'émeraude, de citrine et d'améthyste, et la suspension aqueuse de poudres est obtenue par broyage de malachite, de rhodocrosite et de smithsonite.

7. Composition selon l'une quelconque des revendications précédentes, **caractérisé en ce que** la composition comprend en outre un gélifiant.

8. Composition selon la revendication 7, **caractérisé en ce que** le gélifiant est choisi parmi les polyacrylamides, les copolymères acrylate/acide acrylique ou acrylamide/acide acrylamido propane sulfonique, les dérivés de cellulose, le chitosan, des muco-polysaccharides végétaux, et les argiles.

9. Composition selon la revendication 8, **caractérisé en ce que** le gélifiant est l'hydroxypropyl cellulose ou un mélange de polyacrylamide, C13-14-isoparaffine et Laureth-7.

10. Composition l'une quelconque des revendications précédentes, **caractérisé en ce que** la composition contient en outre du nitrure de bore.

11. Composition selon l'une quelconque des revendications précédente, **caractérisé en ce que** la composition contient en outre une poudre de saphir.

12. Composition selon l'une quelconque des revendications précédente, **caractérisé en ce que** le rapport poudres sèches / suspension de poudres est compris entre 1/5 et 1/1.

13. Composition selon l'une quelconque des revendications précédente, **caractérisé en ce que** la composition comprend entre 1 et 50% en poids de concentré minéral.

14. Composition dermatologique selon l'une quelconque des revendications 1 à 13, destinée à être utilisé pour activer les fonctions microcirculatoires de la peau.

15. Composition dermatologique selon l'une quelconque des revendications 1 à 13, destinée à être utilisée pour protéger la peau contre les rayonnements électromagnétiques.

16. Utilisation non-thérapeutique de la composition cosmétique selon l'une quelconque des revendications 1 à 13, pour protéger la peau.

17. Procédé de préparation d'une composition selon l'une quelconque des revendications 1 à 15, le procédé comprenant les étapes suivantes :
- mélangeur :
- la poudre ou le mélange de poudres sèches, et
- la suspension aqueuse de poudres, et
- former un concentré minéral, en mélange avec un support et des excipients physiologiquement acceptables.

## Patentansprüche

1. Kosmetische und/oder dermatologische Zusammensetzung für den Schutz der Haut, **dadurch gekennzeichnet, dass** sie umfasst:
- ein Pulver oder ein Gemisch trockner Pulver, erhalten aus Edel- oder Halbedelsteinen, enthaltend mindestens ein Siliziumderivat, umfassend ein Siliziumoxid und/oder ein Silikat, wobei die Edel- oder Halbedelsteine aus dem Turmalin, dem Aquamarin, dem Smaragd, dem Citrin, dem Amethyst, dem Lapislazuli und dem Topas ausgewählt sind, und,
- eine wässrige Aufschlämmung von Pulvern, erhalten aus Edel- oder Halbedelsteinen, enthaltend mindestens ein Spurenelement, das kein Silizium ist, wobei die Edel- oder Halbedelsteine aus dem Malachit, dem Rhodocrosit, dem Smithsonit, dem Rubin und dem Saphir ausgewählt sind,
um in Mischung mit einem Träger und physiologisch akzeptablen Hilfsstoffen ein Mineralkonzentrat zu bilden.

2. Zusammensetzung nach Anspruch 1, **dadurch gekennzeichnet, dass** das Spurenelement aus dem Eisen, dem Kupfer, dem Zink, dem Mangan, dem Molybdän, dem Bor, dem Kobalt, dem Aluminium und dem Selen ausgewählt ist.

3. Zusammensetzung nach Anspruch 1 oder 2, **dadurch gekennzeichnet, dass** das Siliziumderivat aus einem Borsilikat, einem Aluminosilikat und dem Siliziumdioxid ausgewählt ist.

4. Zusammensetzung nach einem der vorangehenden Ansprüche, **dadurch gekennzeichnet, dass** das Spurenelement aus dem Eisen, dem Kupfer, dem Zink und dem Mangan ausgewählt ist.

5. Zusammensetzung nach einem der vorangehenden Ansprüche, **dadurch gekennzeichnet, dass** die Korngröße der Pulver zwischen 1 µm und 50 µm inklusive ist.

6. Zusammensetzung nach Anspruch 1, **dadurch gekennzeichnet, dass** das Gemisch trockener Pulver durch Zerkleinern von Aquamarin, Turmalin, Smaragd, Citrin und Amethyst erhalten wird und die wässrige Aufschlämmung von Pulvern durch Zerkleinern von Malachit, Rhodocrosit und Smithsonit erhalten wird.

7. Zusammensetzung nach einem der vorangehenden Ansprüche, **dadurch gekennzeichnet, dass** die Zusammensetzung ferner ein Geliermittel umfasst.

8. Zusammensetzung nach Anspruch 7, **dadurch gekennzeichnet, dass** das Geliermittel aus den Polyacrylamiden, den Copolymeren Acrylat/Acrylsäure oder Acrylamid/Acrylamidopropansulfonsäure, den Derivaten der Cellulose, dem Chitosan, den pflanzlichen Muco-Polysacchariden und den Tonerden ausgewählt ist.

9. Zusammensetzung nach Anspruch 8, **dadurch gekennzeichnet, dass** das Geliermittel Hydroxypropylcellulose oder ein Gemisch aus Polyacrylamid, C₁₃-C₁₄-Isoparaffin und Laureth-7 ist.

10. Zusammensetzung nach einem der vorangehenden Ansprüche, **dadurch gekennzeichnet, dass** die Zusammensetzung ferner Bornitrid enthält.

11. Zusammensetzung nach einem der vorangehenden Ansprüche, **dadurch gekennzeichnet, dass** die Zusammensetzung ferner ein Saphirpulver enthält.

12. Zusammensetzung nach einem der vorangehenden Ansprüche, **dadurch gekennzeichnet, dass** das Verhältnis trockene Pulver / Pulveraufschlämmung zwischen 1/5 und 1/1 inklusive ist.

13. Zusammensetzung nach einem der vorangehenden Ansprüche, **dadurch gekennzeichnet, dass** die Zusammensetzung zwischen 1 und 50 Gew.-% Mineralkonzentrat enthält.

14. Dermatologische Zusammensetzung nach einem der Ansprüche 1 bis 13, die bestimmt ist, zur Aktivierung der Mikrozirkulationsfunktionen der Haut verwendet zu werden.

15. Dermatologische Zusammensetzung nach einem der Ansprüche 1 bis 13, die bestimmt ist, zum Schutz der Haut vor elektromagnetischen Strahlungen verwendet zu werden.

16. Nicht therapeutische Verwendung der kosmetischen Zusammensetzung nach einem der Ansprüche 1 bis 13, um die Haut zu schützen.

17. Verfahren zur Herstellung einer Zusammensetzung nach einem der Ansprüche 1 bis 15, wobei das Verfahren die folgenden Schritte umfasst:
- Mischen :
- das Pulver oder das Gemisch trockener Pulver, und
- die wässrige Pulveraufschlämmung, und
- Bilden eines Mineralkonzentrats in Mischung mit einem Träger und physiologisch akzeptablen Hilfsstoffen.

## Claims

1. A cosmetic and/or dermatological composition for skin protection, **characterized in that** it comprises:
- a powder or mixture of dry powders obtained from precious or semi-precious stones containing at least one silicon derivative comprising a silicon oxide (silica) and/or a silicate, said precious or semi-precious stones being selected from among tourmaline, aquamarine, emerald, citrine, amethyst, lapis lazuli and topaz; and
- an aqueous suspension of powders obtained from precious or semi-precious stones containing at least one trace element other than silicon, said precious or semi-precious stones being selected from among malachite, rhodocrosite, smithsonite, ruby and sapphire;
to form a mineral concentrate in a mixture with a carrier and excipients that are physiologically acceptable.

2. The composition according to claim 1, **characterized in that** the trace element is selected from among iron, copper, zinc, manganese, molybdenum, boron, cobalt, aluminium and selenium.

3. The composition according to claim 1 or 2, **characterized in that** the silicon derivative is selected from among a borosilicate, aluminosilicate and silicon dioxide.

4. The composition according to any of the preceding claims, **characterized in that** the trace element is selected from among iron, copper, zinc and manganese.

5. The composition according to any of the preceding claims, **characterized in that** the particle size of the powders is between 1 µm and 50 µm.

6. The composition according to claim 1, **characterized in that** the mixture of dry powders is obtained by grinding aquamarine, tourmaline, emerald, citrine and amethyst, and the aqueous suspension of powders is obtained by grinding malachite, rhodocrosite and smithsonite.

7. The composition according to any of the preceding claims, **characterized in that** the composition further comprises a gelling agent.

8. The composition according to claim 7, **characterized in that** the gelling agent is selected from among polyacrylamides, acrylate/acrylic acid or acrylamide/acrylamido-propane sulfonic acid copolymers, cellulose derivatives, chitosan, plant mucopolysaccharides and clays.

9. The composition according to claim8, **characterized in that** the gelling agent is hydroxypropylcellulose or a mixture of polyacrylamide, C13-14-isoparaffin and Laureth-7.

10. The composition according to any of the preceding claims, **characterized in that** the composition further contains boron nitride.

11. The composition according to any of the preceding claims, **characterized in that** the composition further contains a sapphire powder.

12. The composition according to any of the preceding claims, **characterized in that** the ratio of dry powders/suspension of powders is between 1:5 and 1:1.

13. The composition according to any of the preceding claims, **characterized in that** the composition comprises between 1 and 50 % by weight of mineral concentrate.

14. A dermatological composition according to any of claims 1 to 13, intended to be used to activate the microcirculatory functions of the skin.

15. A dermatological composition according to any of claims 1 to 13, intended to be used to protect the skin against electromagnetic radiation.

16. The non-therapeutic use of the cosmetic composition according to any of claims 1 to 13, for skin protection

17. A method to prepare a composition according to any of claims 1 to 15, the method comprising the following steps:
• mixing:
- the powder or mixture of dry powders, and
- the aqueous suspension of powders; and
• forming a mineral concentrate in a mixture with a carrier and excipients that are physiologically acceptable.
